# EUROPÄISCHE PATENTSCHRIFT

(11) **EP 0 444 508 B1**
(45) Veröffentlichungstag und Bekanntmachung des Hinweises auf die Patenterteilung: **13.09.1995**
(21) Anmeldenummer: 91102407.3
(22) Anmeldetag: 20.02.1991
(51) Int. Cl.: C07C 269/02, C07C 271/12, C07C 271/20, C07C 271/28

(54) **Ethylenisch ungesättigte, fluorhaltige Urethanderivate und Verfahren zu ihrer Herstellung**
Ethylenically unsaturated fluorine-containing urethan derivatives and process for their preparation
Dérivés d'uréthane insaturés éthyléniquement contenant du fluor et procédé de leur préparation

(30) Priorität: 27.02.1990 DE 4006097
(43) Veröffentlichungstag der Anmeldung: 04.09.1991
(73) Patentinhaber: HOECHST AKTIENGESELLSCHAFT, 65926 Frankfurt am Main (DE)
(72) Erfinder: Huth, Hans-Ullrich, Dr., W-6073 Egelsbach (DE)

(56) Entgegenhaltungen:
- EP-A- 0 040 923
- EP-A- 0 181 281
- EP-A- 0 225 826
- EP-A- 0 308 801

## Beschreibung

Die Erfindung betrifft ethylenisch ungesättigte, fluorhaltige Urethanderivate, deren Urethangruppe an ihrem Carboxyrest durch einen fluorierten Alkyl-, Alkoxyalkyl-, Aryloxyalkyl-, Alkylaryloxyalkyl- oder Arylalkoxyalkylrest und an ihrem N-Atom durch einen nichtfluorierten, ethylenisch ungesättigten organischen Rest substituiert ist und die sich u.a. radikalisch polymerisieren bzw. copolymerisieren lassen, sowie Verfahren zu ihrer Herstellung.

Aus der EP-PS 24908 ist die Herstellung von ethylenisch ungesättigten, fluorhaltigen Urethanen, deren Urethangruppe am N-Atom einen Fluoralkylrest besitzen kann, bekannt. Polymere aus diesen Urethanen sollen in ungiftigen Lösungsmitteln löslich und als schmutzabweisende Appretur mit guter Haftung auf Textilien und Leder und hoher Erweichungstemperatur verwendbar sein.

Aus der US-PS 4540805 ist die Herstellung von ethylenisch ungesättigten, fluorhaltigen Urethanen, deren Urethangruppe am Carboxyrest durch fluorierte Thioätherreste substituiert ist, bekannt. Die Verbindungen können durch Polymerisation in Substanz, in Lösung oder in Emulsion in Polymere übergeführt werden, die u.a. zur wasser- und ölabweisenden Beschichtung von Textilien verwendbar sein sollen.

Aus der EP-PS 225826 ist die Herstellung von ethylenisch ungesättigten, fluorhaltigen Bisurethanen, die durch stufenweise Umsetzung von zunächst einer Isocyanatgruppe des Toluylendiisocyanats mit einem Fluoralkanol und danach der anderen Isocyanatgruppe mit einem Hydroxyalkyl(meth-) acrylat erhalten werden können, bekannt. Das Verfahren hat den Nachteil, daß bereits in der ersten Stufe bei bis zu 40% des Toluylendiisocyanats beide Isocyanatgruppen mit Fluoralkanol reagieren, was zu erheblichen Verlusten führt und kostspielige Trennoperationen erforderlich macht. Durch Lösungspolymerisation hergestellte Homo- und Copolymere der fluorhaltigen Bisurethane sollen zur Herstellung von wasser- und ölabweisenden Beschichtungen auf Textilien und Leder verwendet werden können.

Wie sich in der Praxis gezeigt hat, sind die bisher bekannt gewordenen ethylenisch ungesättigten, fluorhaltigen Urethane mit einer Reihe von Nachteilen behaftet, wie z.B. schwierig und aufwendig zugänglichen Ausgangskomponenten, unbefriedigenden Ausbeuten bei den Herstellungsverfahren, Erfordernis von aufwendigen Reinigungsoperationen zur Eliminierung von Nebenprodukten, unbefriedigenden Löslichkeiten in Lösungsmitteln bei der Weiterverwendung der Monomeren als Zwischenprodukte, unbefriedigendem Homo- und/oder Copolymerisationsverhalten sowie unzureichendem Eigenschaftsspektrum von Polymerisaten und/oder Copolymerisaten.

Der Erfindung lag somit die Aufgabe zugrunde, unter Überwindung der vorgenannten Schwierigkeiten monomere, ethylenisch ungesättige, fluorhaltige Urethane verfügbar zu machen, die einfach und wirtschaftlich herstellbar sind und deren Eigenschaftsspektrum eine breite und unproblematische Verwendbarkeit ermöglicht.

Es wurden nun überraschenderweise ethylenisch ungesättigte, fluorhaltige Urethanderivate gefunden, deren Urethangruppe an ihrem Carboxyrest durch einen fluorierten organischen Rest und an ihrem N-Atom durch einen nichtfluorierten, ethylenisch ungesättigten organischen Rest substituiert ist, ferner Verfahren zu ihrer Herstellung. Die erfindungsgemäßen Urethanderivate sind unter Normalbedingungen flüssig oder fest, besitzen gute Löslichkeiten in den üblicherweise zu ihrer Weiterverwendung benötigten organischen Lösungsmitteln und lassen sich vorteilhaft und vielseitig, z.B. zur Herstellung von Polymerisaten oder Copolymerisaten, verwenden.

Gegenstand der Erfindung sind daher ethylenisch ungesättigte, fluorhaltige Urethanderivate der Formel I, 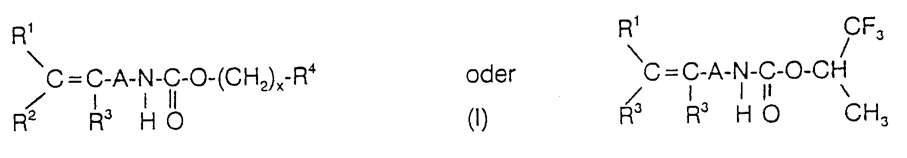
worin R¹ bis R⁴, A und der Zahlenindex x folgendes bedeuten:
- R¹, R², R³,: die gleich oder verschieden sein können,
= H, -CH₃, vorzugsweise R¹, R² = H und R³ = -CH₃,
- x: = 1 oder 2,
- A: = -(CH₂)_{y}-, y = 1 bis 6,
tert.-Butylphenylen -[C₆H₃-C(CH₃)₃]- oder
-C₆H₄C(CH₃)₂- oder 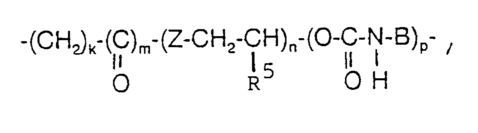
- Z: = Sauerstoff oder NH, vorzugsweise Sauerstoff,
- R⁵: = H, -CH₃, -C₂H₅,
- B: = (C₆-C₁₆)-Arylen, das (C₁-C₁₀)-Alkylreste enthalten kann,
vorzugsweise Phenylen, Toluylen, tert.-Butylphenylen, Naphthylen, (C₂-C₁₂)-Alkylen oder (C₆-C₁₀)-Cycloalkylen,
- k, m, p: = 0 oder 1, vorzugsweise m = 1,
- n: = 1 bis 5, vorzugsweise 1,
- R⁴: = -CF₂-CFH-CF₃ oder CFH-CF₂-CF₂-CF₃.

Besonders bevorzugte Urethanderivate sind solche, bei denen in Formel I R¹, R² = H, R³ = H, -CH₃ oder R¹, R³ = H, R² = -CH³, und Z = Sauerstoff bedeutet.

Gegenstand der Erfindung ist ferner ein Verfahren zur Herstellung von ethylenisch ungesättigten, fluorhaltigen Urethanderivaten der Formel I, 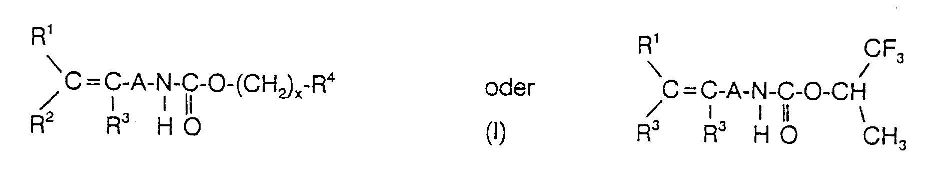
worin R¹ bis R⁴, A und x die vorstehend genannten Bedeutungen haben, durch Umsetzung von Isocyanaten mit organischen Hydroxyverbindungen, indem man Isocyanate der Formel II, 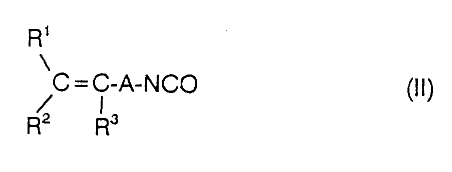
worin R1 bis R3 und A die Bedeutung wie in Formel I haben, mit äquimolaren Mengen von fluorierten Alkanolen aus der Gruppe
1,1,1,3,3,3-Hexafluorpropanol-2,
2,2,3,4,4,4-Hexafluorbutanol-1,
2,3,3,4,4,5,5,5-Oktafluorpentanol-1 umsetzt.

Bevorzugte Ausgangskomponenten der Formel II sind beispielsweise die folgenden ethylenisch ungesättigten Verbindungen mit endständiger isocyanatgruppe: Isocyanatoethylacrylat, Isocyanatoethylmethacrylat, Isocyanatopropylmethacrylat, Isocyanatopropylacrylat, Isocyanatoethylcrotonat, o-Isopropenyl-α,α-dimethylbenzylisocyanat, m-Isopropenyl-α,α-dimethylbenzylisocyanat, p-Isopropenyl-α,α-dimethylbenzylisocyanat, Vinylisocyanat, Propenylisocyanat,
Isocyanatotoluylcarbamidsäure-(2-methacryloyloxy-ethyl)-ester,
Isocyanatotoluylcarbamidsäure-(2-acryloyloxy-ethyl)-ester,
Isocyanatotoluylcarbamidsäure-(3-methacryloyloxy-propyl)-ester,
Isocyanatotoluylcarbamidsäure-(3-acryloyloxy-propyl)-ester,
Isocyanatotoluylcarbamidsäure-(2-crotonoyloxy-ethyl)-ester,
6-Isocyanato-hexylcarbamidsäure-(2-methacryloyloxy-ethyl)-ester,
6-Isocyanato-hexylcarbamidsäure-(2-acryloyloxy-ethyl)-ester,
6-Isocyanato-hexylcarbamidsäure-(3-methacryloyloxy-propyl)-ester,
6-Isocyanato-hexylcarbamidsäure-(3-acryloyloxy-propyl)-ester,
6-Isocyanato-hexylcarbamidsäure-(2-crotonoyloxy-ethyl)-ester,
(5-Isocyanato-1,3,3-trimethyl-cyclohexyl)-methylcarbamidsäure-(2-methacryloyloxy-ethyl)-ester,
(5-Isocyanato-1,3,3-trimethyl-cyclohexyl)-methylcarbamidsäure-(2-acryloyloxy-ethyl)-ester,
(5-Isocyanato-1,3,3-trimethyl-cyclohexyl)-methylcarbamidsäure-(3-methacryloyloxy-propyl)-ester,
(5-Isocyanato-1,3,3-trimethyl-cyclohexyl)-methylcarbamidsäure-(3-acryloyloxy-propyl)-ester,
(5-Isocyanato-1,3,3-trimethyl-cyclohexyl)-methylcarbamidsäure(2-crotonoyloxy-ethyl)-ester,
(3-Isocyanatomethyl-3,5,5-trimethyl-cyclohexyl)-carbamidsäure-(2-methacryloyloxy-ethyl)-ester,
(3-Isocyanatomethyl-3,5,5-trimethyl-cyclohexyl)-carbamidsäure-(2-acryloyloxy-ethyl)-ester,
(3-Isocyanatomethyl-3,5,5-trimethyl-cyclohexyl)-carbamidsäure-(3-methacryloyloxy-propyl)-ester,
(3-Isocyanatomethyl-3,5,5-trimethyl-cyclohexyl)carbamidsäure-(3-acryloyloxy-propyl)-ester, (3-Isocyanatomethyl-3,5,5-trimethyl-cyclohexyl)carbamidsäure-(2-crotonoxyloxyethyl)ester.

Die Durchführung des Verfahrens erfolgt erfindungsgemäß bevorzugt in der Weise, daß man die Umsetzung in Substanz oder in inerten organischen Lösungsmitteln oder gegebenenfalls in unter den Umsetzungsbedingungen sich inert verhaltenden copolymerisationsfähigen ethylenisch ungesättigten Monomeren (Reaktivverdünner) in Abwesenheit von Wasser, vorzugsweise bei Temperaturen zwischen 0 °C und 100 °C, insbesondere zwischen 0 und 80 °C, durchführt.

Als inerte organische Lösungsmittel kommen die bei organischen Synthesen mit Isocyanaten in wasserfreiem Medium üblicherweise verwendeten inerten Lösungsmittel zur Anwendung, sofern sie u.a. die Erfordernisse hinsichtlich Lösewirkung und Siedebereich erfüllen können.

Bevorzugte inerte Lösungsmittel sind z.B. Toluol, Tetrahydrofuran (THF), Ethylacetat und Hexan.

In manchen Fällen können ferner auch sogenannte Reaktivverdünner als Lösungsmittel vorteilhaft sein, das sind copolymerisationsfähige Monomere, die sich unter den Synthesebedingungen der erfindungsgemäßen Urethangruppenbildung inert verhalten, sich aber später unter geeigneten Polymerisationsbedingungen mit den erfindungsgemäßen ungesättigten Urethanderivaten der Formel I copolymerisieren lassen. Bevorzugte inerte Reaktivverdünner sind demzufolge z.B. (Meth-)Acrylsäureester, Styrol und Vinylester, wobei man bei deren Verwendung vorzugsweise unterhalb der Sättigungskonzentration der Reaktanten arbeitet.

Gegebenenfalls wird bei der erfindungsgemäßen Isocyanataddition ein Katalysator mitverwendet, u.a. um die Reaktionstemperatur möglichst niedrig halten zu können, was insbesondere bei der Verwendung von Reaktivverdünnern vorteilhaft sein kann. Bevorzugte Katalysatoren sind organische Zinnverbindungen, die vorzugsweise in einem inerten organischen Lösungsmittel gelöst eingesetzt werden.

Besonders bevorzugt ist die Verwendung von Dibutylzinndilaurat, gegebenenfalls in Kombination mit tert.-Butylbrenzkatechin, ferner beispielsweise von tert.-Aminen, wie z.B. Dimethylpiperazin, 1,4-Diazobicyclo(2,2,2)oktan.

Die erfindungsgemäß hergestellten Verbindungen der Formel I werden in den meisten Fällen unmittelbar in flüssiger oder hochviskoser oder wachsartiger oder fester bzw. kristalliner Form erhalten und können, gegebenenfalls nach Eliminierung von mitverwendeten Lösungsmitteln, im allgemeinen ohne weitere Reinigung und Trocknung als Zwischenprodukte in Substanz oder gegebenenfalls gelöst in organischen Lösungsmitteln oder emulgiert bzw. dispergiert in Wasser weiter verwendet werden.
Selbstverständlich können die bei dem erfindungsgemäßen Syntheseverfahren anfallenden rohen Reaktionsprodukte auch nach üblichen Methoden, wie z.B. Eluieren, Umkristallisieren, Umfällen und/oder Abdestillieren flüchtiger Bestandteile, gegebenenfalls unter vermindertem Druck und gegebenenfalls unter Mitverwendung üblicher Polymerisationsinhibitoren sowie gegebenenfalls unter Schutzgas, gereinigt und die Verbindungen der Formel I in chemisch reiner Form gewonnen werden.

Die fluorhaltigen Verbindungen der Formel I sind polymerisationsfähig und copolymerisationsfähig. Sie sind im allgemeinen gut bis sehr gut löslich in üblichen nichtwäßrigen, unpolaren, inerten organischen Lösungsmitteln sowie auch in den oben bereits erwähnten sogenannten Reaktivverdünnern und eignen sich insbesondere als Monomere bzw. Comonomere für Polymerisationen. Es wurde diesbezüglich überraschenderweise gefunden, daß vorzugsweise durch radikalisch initiierte Emulsions- oder Suspensionspolymerisation hergestellte Polymerisate aus Monomeren der Formel I sowie insbesondere Copolymerisate auf der Basis von ethylenisch ungesättigten Monomeren, die mindestens 0,1 Gew.-% Monomereinheiten aus fluorhaltigen Urethanderivaten der Formel I der vorliegenden Erfindung enthalten, unerwartet vorteilhafte Eigenschaften besitzen.

Diese Copolymerisate, ihre Verwendbarkeit, insbesondere in Form wäßriger Copolymerisatdispersionen, sowie ihre Herstellung unter Verwendung monomerer fluorhaltiger Urethanderivate der Formel I der vorliegenden Erfindung als Ausgangsprodukte sind u.a. Gegenstand der am gleichen Tag eingereichten Patentanmeldung EP-A 0 444 509, auf die hiermit Bezug genommen wird.

Die Erfindung wird durch die nachstehenden Beispiele näher erläutert, ohne sie dadurch zu beschränken.

### Beispiel 1-3

Herstellung von ethylenisch ungesättigten fluorhaltigen Urethanderivaten der Formel I durch Umsetzung äquimolarer Mengen der Ausgangskomponenten.

### Beispiel 1

In einem 0,25 l Vierhalskolben mit Rührer, Thermometer, Tropftrichter und Rückflußkühler mit CaCl₂-Endrohr werden 0,69 mol 2,2,3,4,4,4-Hexafluorbutanol-1 und 2 Tropfen Dibutylzinndilaurat vorgelegt und auf 50 °C erwärmt. Unter Rühren werden dieser Mischung sodann 0,69 mol des Formel II-Isocyanats 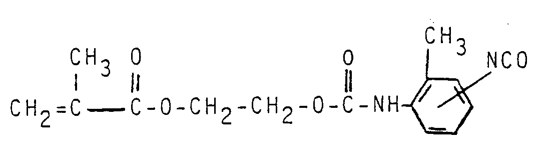
innerhalb von 45 Minuten zugetropft, wobei die Innentemperatur der Mischung auf 58 °C ansteigt. Anschließend wird die Mischung bei 80 °C so lange weitergerührt, bis die NCO-Bande im IR-Spektrum des Reaktionsgemisches verschwunden ist (Dauer ca. 2 Tage). Beim anschließenden Abkühlen der Mischung kristallisiert der Kolbeninhalt vollständig aus. Er wird in einer Reibschale zerkleinert, mit insgesamt 88 ml Cyclohexan gewaschen und unter Vakuum getrocknet. Es wird das entsprechende ethylenisch ungesättigte, fluorhaltige Urethanderivat der Formel I in fester Form und heller Farbe (Ausbeute 95 % der Theorie) mit einem Schmelzpunkt Fp.(Kapillarmethode) von 84-87 °C erhalten.

### Beispiel 2

Das Beispiel 1 wird unter Reaktantenaustausch wiederholt mit der Abänderung, daß als Reaktanten 0,69 mol 2,2,3,4,4,4-Hexafluorbutanol-1 und 0,69 mol des Formel II-Isocyanats 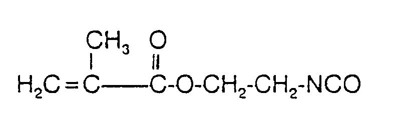
eingesetzt werden. Es wird ohne Cyclohexanwäsche gearbeitet und in praktisch 100 %iger Ausbeute das entsprechende ethylenisch ungesättigte Urethanderivat der Formel I in flüssiger, farbloser Form erhalten. Der Siedepunkt bei Normaldruck liegt bei über 160 °C unter Zersetzung.

### Beispiel 3

Das Beispiel 1 wird unter Reaktantenaustausch wiederholt mit der Abänderung, daß als Reaktanten 0,69 mol 1,1,1,3,3,3-Hexafluorpropanol-2 und 0,69 mol des Formel II-Isocyanats 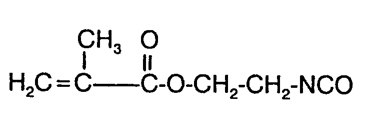
eingesetzt werden. Es wird das entsprechende ethylenisch ungesättigte, fluorhaltige Urethanderivat der Formel I nach erfolgter Cyclohexanwäsche in 95 %iger Ausbeute in fester Form und heller Farbe erhalten. Der Schmelzpunkt Fp. (Kapillarmethode) beträgt 55 °C.

## Patentansprüche (Patentansprüche für folgende(n) Vertragsstaat(en): AT, BE, CH, DE, FR, GB, GR, IT, LI, NL, SE)

1. Ethylenisch ungesättigte, fluorhaltige Urethanderivate der Formel I, 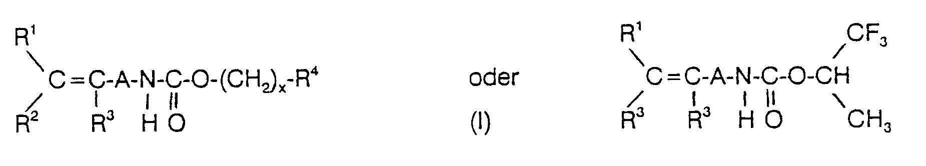 worin R¹ bis R⁴, A und der Zahlenindex x folgendes bedeuten:
R¹, R², R³, die gleich oder verschieden sein können,
= H, -CH₃, vorzugsweise R¹, R² = H und R³ = -CH₃,
x = 1 oder 2,
A = -(CH₂)_{y}-, y = 1 bis 6,
tert.-Butylphenylen -[C₆H₃-C(CH₃)₃]- oder
-C₆H₄C(CH₃)₂- oder 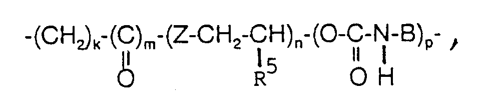
Z = Sauerstoff oder NH, vorzugsweise Sauerstoff,
R⁵ = H, -CH₃, -C₂H₅,
B = (C₆-C₁₆)-Arylen, das (C₁-C₁₀)-Alkylreste enthalten kann,
vorzugsweise Phenylen, Toluylen, tert.-Butylphenylen, Naphthylen, (C₂-C₁₂)-Alkylen oder (C₆-C₁₀)-Cycloalkylen,
k, m, p = 0 oder 1, vorzugsweise m = 1,
n = 1 bis 5, vorzugsweise 1,
R⁴ = -CF₂-CFH-CF₃ oder CFH-CF₂-CF₂-CF₃.

2. Urethanderivate nach Anspruch 1, dadurch gekennzeichnet, daß in Formel I R¹, R² = H, R³ = H, -CH₃ oder R¹, R³ = H, R² = -CH₃, Z = Sauerstoff bedeuten.

3. Verfahren zur Herstellung von ethylenisch ungesättigten fluorhaltigen Urethanderivaten der Formel I nach Anspruch 1 und/oder 2, 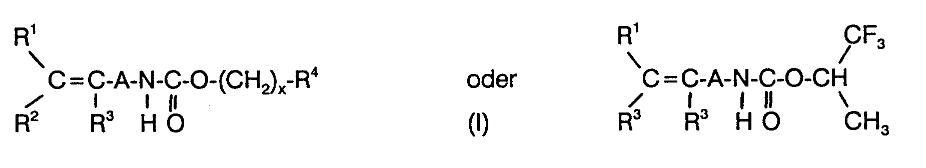 worin R¹ bis R⁴, A und x die Bedeutung wie in Formel I in Anspruch 1 und/oder 2 haben, durch Umsetzung von Isocyanaten mit organischen Hydroxyverbindungen, dadurch gekennzeichnet, daß man Isocyanate der Formel II, 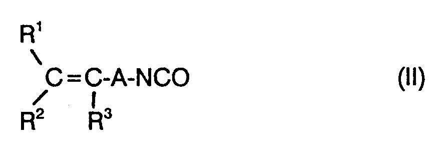 worin R¹ bis R³ und A die Bedeutung wie in Formel I haben, mit äquimolaren Mengen von fluorierten Alkanolen aus der Gruppe
1,1,1,3,3,3-Hexafluorpropanol-2,
2,2,3,4,4,4-Hexafluorbutanol-1,
2,3,3,4,4,5,5,5-Oktafluorpentanol-1
umsetzt.

4. Verfahren nach Anspruch 3, dadurch gekennzeichnet, daß man die Umsetzung in Substanz oder in inerten organischen Lösungsmitteln bzw. in unter den Umsetzungsbedingungen sich inert verhaltenden copolymerisationsfähigen ethylenisch ungesättigten Monomeren in Abwesenheit von Wasser, vorzugsweise bei Temperaturen zwischen 0 °C und 100 °C durchführt.

## Patentansprüche (Patentansprüche für folgende(n) Vertragsstaat(en): ES)

1. Verfahren zur Herstellung von ethylenisch ungesättigten fluorhaltigen Urethanderivaten der Formel I, 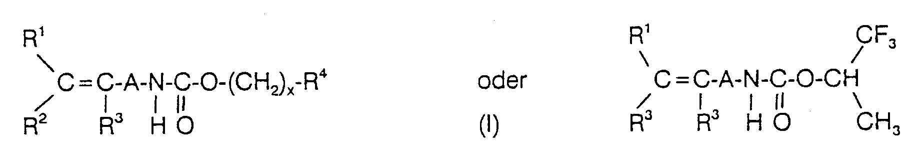 worin R¹ bis R⁴, A und der Zahlenindex x folgendes bedeuten:
R¹, R², R³, die gleich oder verschieden sein können,
= H, -CH₃, vorzugsweise R¹, R² = H und R³ = -CH₃,
x = 1 oder 2 ,
A = -(CH₂)_{y}-, y = 1 bis 6, tert. Butylphenylen -[C₆H₃-C(CH₃)₃]-
oder -C₆H₄C(CH₃)₂-
oder 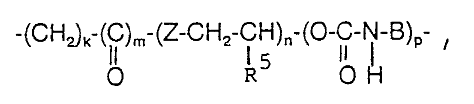
Z = Sauerstoff oder NH, vorzugsweise Sauerstoff,
R⁵ = H, -CH₃, -C₂H₅,
B = (C₆-C₁₆)-Arylen, das (C₁-C₁₀)-Alkylreste enthalten kann,
vorzugsweise Phenylen, Toluylen, tert.-Butylphenylen, Naphthylen, (C₂-C₁₂)-Alkylen oder (C₆-C₁₀)-Cycloalkylen,
k, m, p, = 0 oder 1, vorzugsweise m = 1,
n = 1 bis 5, vorzugsweise 1,
R⁴ = -CF₂-CFH-CF₃ oder -CFH-CF₂-CF₂-CF₃,
durch Umsetzung von Isocyanaten mit organischen Hydroxyverbindungen, dadurch gekennzeichnet, daß man Isocyanate der Formel II, 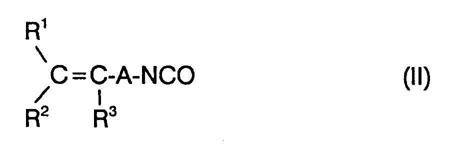 worin R¹ bis R³ und A die Bedeutung wie in Formel I haben, mit äquimolaren Mengen von fluorierten Alkanolen aus der Gruppe
1,1,1,3,3,3-Hexafluorpropanol-2,
2,2,3,4,4,4-Hexafluorbutanol-1,
2,3,3,4,4,5,5,5-Oktafluorpentanol-1
umsetzt.

2. Verfahren nach Anspruch 1, dadurch gekennzeichnet, daß man die Umsetzung in Substanz oder in inerten organischen Lösungsmitteln bzw. in unter den Umsetzungsbedingungen sich inert verhaltenden copolymerisationsfähigen ethylenisch ungesättigten Monomeren in Abwesenheit von Wasser, vorzugsweise bei Temperaturen zwischen 0 °C und 100 °C durchführt.

3. Verfahren nach Anspruch 1 oder 2, dadurch gekennzeichnet, daß in Formel I R¹, R² = H, R³ = H, -CH₃ oder R¹, R³ = H, R² = -CH₃, Z = Sauerstoff bedeutet.

## Claims (Claims for the following Contracting State(s): AT, BE, CH, DE, FR, GB, GR, IT, LI, NL, SE)

1. Ethylenically unsaturated, fluorine-containing urethane derivatives of the Formula I, 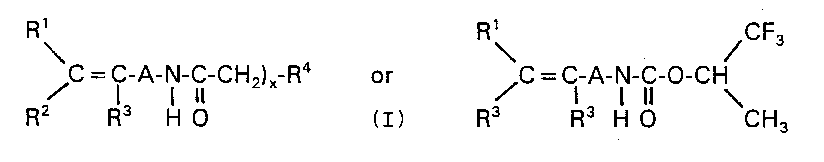 in which R¹ to R⁴, A and the numerical index x have the following meanings:
R¹, R² and R³, which may be identical or different,
are H, -CH₃, R¹ and R² preferably being H and R³ preferably being CH₃
x is 1 or 2
A is -(CH₂)_{y}- (y being 1 to 6),
tert.-butylphenylene-[C₆H₃-C(CH₃)₃-]-or -C₆H₄C(CH₃)₂- or 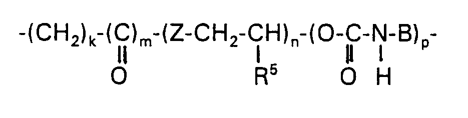
where
Z is oxygen or NH, preferably oxygen,
R⁵ is H, -CH₃, -C₂H₅,
B is (C₆-C₁₆)arylene which may contain
(C₁-C₁₀)alkyl radicals, preferably phenylene, toluylene, tert.-butylphenylene, naphthylene,
(C₂-C₁₂)alkylene or
(C₆-C₁₀)cycloalkylene,
k, m, p are 0 or 1, m preferably being 1
n is 1 to 5, preferably 1, and
R⁴ is -CF₂CFH-CF₃ or -CFH-CF₂-CF₂-CF₃.

2. Urethane derivatives as claimed in claim 1, wherein in Formula I, R¹ and R² are H, R³ is H or -CH₃, or R¹ and R³ are H, R² is CH₃ and Z is oxygen.

3. A process for the preparation of ethylenically unsaturated, fluorine-containing urethane derivatives of the Formula I as claimed in claim 1 and/or 2 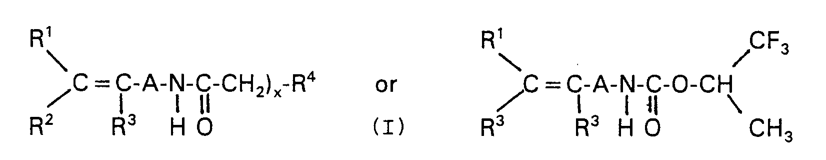 in which R¹ to R⁴, A and x have the meaning as in Formula I in claim 1 and/or 2, by reacting isocyanates with organic hydroxyl compounds, which process comprises reacting isocyanates of the Formula II, 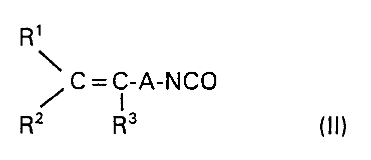 in which R¹ to R³ and A have the meaning as in Formula I, with equimolar amounts of fluorinated alkanols selected from the group comprising
1,1,1,3,3,3-hexafluoropropanol-2,
2,2,3,4,4,4-hexafluorobutanol-1, and
2,3,3,4,4,5,5,5-octafluoropentanol-1.

4. The process as claimed in claim 3, wherein the reaction is carried out in bulk or in inert organic solvents or in ethylenically unsaturated monomers capable of copolymerization which behave inertly under the reaction conditions, in the absence of water, preferably at temperatures between 0 °C and 100 °C.

## Claims (Claims for the following Contracting State(s): ES)

1. A process for the preparation of ethylenically unsaturated, fluorine-containing urethane derivatives of the Formula I, 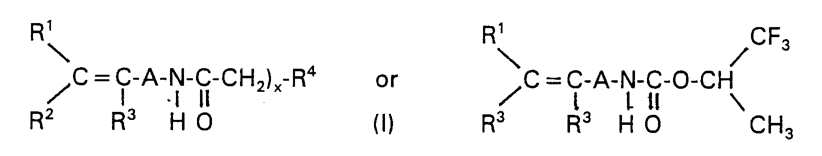 in which R¹ to R⁴, A and the numerical index x have the following meanings:
R¹, R² and R³, which may be identical or different,
are H, -CH₃, R¹ and R² preferably being H and R³ preferably being CH₃
x is 1 or 2
A is -(CH₂)_{y}- (y being 1 to 6),
tert.-butylphenylene-[C₆H₃-C(CH₃)₃-]-or -C₆H₄C(CH₃)₂- or 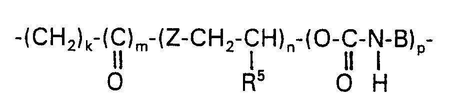
where
Z is oxygen or NH, preferably oxygen,
R⁵ is H, -CH₃, -C₂H₅,
B is (C₆-C₁₆)arylene which may contain
(C₁-C₁₀)alkyl radicals, preferably phenylene, toluylene, tert.-butylphenylene, naphthylene,
(C₂-C₁₂)alkylene or
(C₆-C₁₀)cycloalkylene,
k, m, p are 0 or 1, m preferably being 1
n is 1 to 5, preferably 1, and
R⁴ is -CF₂CFH-CF₃ or -CFH-CF₂-CF₂-CF₃
by reacting isocyanates with organic hydroxyl compounds, which process comprises reacting isocyanates of the Formula II, 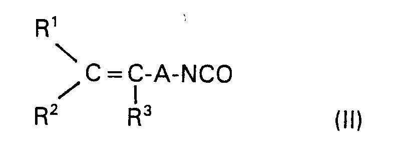 in which R¹ to R³ and A have the meaning as in Formula 1, with equimolar amounts of fluorinated alkanols selected from the group comprising
1,1,1,3,3,3-hexafluoropropanol-2,
2,2,3,4,4,4-hexafluorobutanol-1, and
2,3,3,4,4,5,5,5-octafluoropentanol-1.

2. The process as claimed in claim 1, wherein the reaction is carried out in bulk or in inert organic solvents or in ethylenically unsaturated monomers capable of copolymerization which behave inertly under the reaction conditions, in the absence of water, preferably at temperatures between 0 °C and 100 °C.

3. The process as claimed in claim 1 or 1, wherein in Formula I, R¹ and R² are H, R³ is H or -CH₃, or R¹ and R³ are H, R² is CH₃ and Z is oxygen.

## Revendications (Revendications pour l'(les) Etat(s) contractant(s) suivant(s): AT, BE, CH, DE, FR, GB, GR, IT, LI, NL, SE)

1. Dérivés d'uréthanne fluorés à insaturation éthylénique de formule I 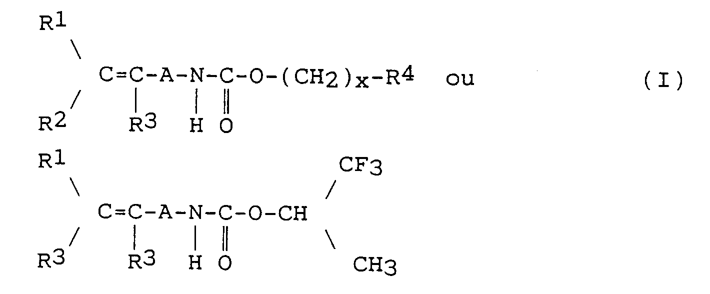 où R¹ à R⁴, A et l'indice numérique x ont les significations suivantes :
R¹, R² et R³, qui peuvent être identiques ou différents, sont chacun H, -CH₃, et de préférence R¹, R² = H et R³ = -CH₃,
x vaut 1 ou 2,
A est -(CH₂)_{y}-, avec y = 1 à 6, le radical tert-butylphénylène -[C₆H₃-C(CH₃)₃]- ou -C₆H₄C(CH₃)₂- ou 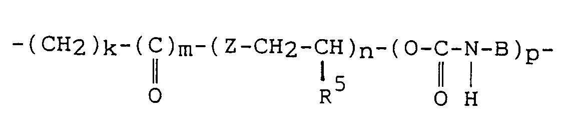 Z est un oxygène ou NH, de préférence un oxygène,
R⁵ est H, -CH₃, -C₂H₅,
B est un radical arylène en C₆-C₁₆ pouvant contenir des radicaux alkyle en C₁-C₁₀, de préférence les radicaux phénylène, toluylène, tert-butylphénylène, naphtylène, alkylène en C₂-C₁₂ ou cycloalkylène en C₆-C₁₀,
k, m, p valent 0 ou 1 et de préférence m = 1,
n vaut de 1 à 5, de préférence 1,
R⁴ est -CF₂-CFH-CF₃ ou CFH-CF₂-CF₂-CF₃.

2. Dérivés d'uréthanne selon la revendication 1, caractérisés en ce que dans la formule I R¹, R² = H, R³ = H, -CH₃ ou R¹, R³ = H, R² = -CH³ et Z est un oxygène.

3. Procédé pour préparer des dérivés d'uréthanne fluorés à insaturation éthylénique de formule I selon la revendication 1 et/ou 2 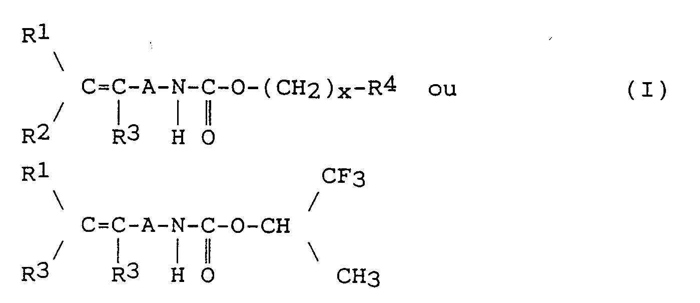 où R¹ à R⁴, A et x ont les significations données dans la formule I de la revendication 1 et/ou 2, par réaction d'isocyanates avec des composés hydroxylés organiques, caractérisé en ce qu'on fait réagir des isocyanates de formule II 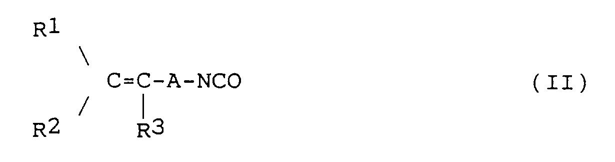 dans laquelle R¹ à R³ et A ont les significations correspondant à la formule I, avec des quantités équimolaires d'alcanols fluorés choisis parmi l'ensemble comprenant le 1,1,1,3,3,3-hexafluoropropanol-2, le 2,2,3,4,4,4-hexafluorobutanol- 1 et le 2,3,3,4,4,5,5,5-octafluoropentanol-1.

4. Procédé selon la revendication 3, caractérisé en ce qu'on met en oeuvre la réaction en l'état ou dans des solvants organiques inertes, ou dans des monomères à insaturation éthylénique, copolymérisables, ayant un comportement inerte dans les conditions de la réaction, en l'absence d'eau, et de préférence à des températures de 0 à 100°C.

## Revendications (Revendications pour l'(les) Etat(s) contractant(s) suivant(s): ES)

1. Procédé pour préparer des dérivés d'uréthanne fluorés à insaturation éthylénique de formule I 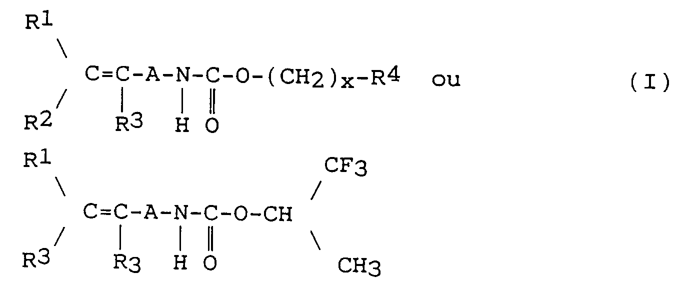 où R¹ à R⁴, A et l'indice numérique x ont les significations suivantes :
R¹, R² et R³, qui peuvent être identiques ou différents, sont chacun H, -CH₃ et de préférence R¹, R² = H et R³ = -CH₃,
x vaut 1 ou 2,
A est -(CH₂)_{y}-, avec y = 1 à 6, le radical tertbutylphénylène -[C₆H₃-C(CH₃)₃]- ou -C₆H₄C(CH₃)₂- ou 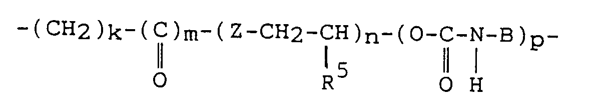 Z est un oxygène ou NH, de préférence un oxygène,
R⁵ est H, -CH₃, -C₂H₅,
B est un radical arylène en C₆-C₁₆ pouvant contenir des radicaux alkyle en C₁-C₁₀, de préférence les radicaux phénylène, toluylène, tert-butylphénylène, naphtylène, alkylène en C₂-C₁₂ ou cycloalkylène en C₆-C₁₀,
k, m, p valent 0 ou 1 et de préférence m = 1,
n vaut de 1 à 5, de préférence 1,
R⁴ est -CF₂-CFH-CF₃ ou CFH-CF₂-CF₂CF₃_{,}
par réaction d'isocyanates avec des composés hydroxylés organiques, caractérisé en ce qu'on fait réagir des isocyanates de formule II 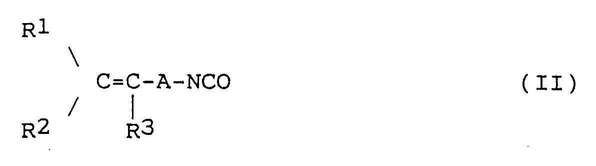 dans laquelle R¹ à R³ et A ont les significations correspondant à la formule I, avec des quantités équimolaires d'alcanols fluorés choisis parmi l'ensemble comprenant le 1,1,1,3,3,3-hexafluoropropanol-2, le 2,2,3,4,4,4-hexafluorobutanol-1 et le 2,3,3,4,4,5,5,5-octafluoropentanol-1.

2. Procédé selon la revendication 1, caractérisé en ce qu'on met en oeuvre la réaction en l'état ou dans des solvants organiques inertes, ou dans des monomères à insaturation éthylénique, copolymérisables, ayant un comportement inerte dans les conditions de la réaction, en l'absence d'eau, et de préférence à des températures de 0 à 100°C.

3. Procédé selon la revendication 1 ou 2, caractérisé en ce que dans la formule I R¹, R² = H, R³ = H, -CH₃ ou R¹, R³ = H, R² = -CH³ et Z est un oxygène.
